# EUROPÄISCHE PATENTSCHRIFT

(11) **EP 0 337 509 B1**
(45) Veröffentlichungstag und Bekanntmachung des Hinweises auf die Patenterteilung: **03.11.1993**
(21) Anmeldenummer: 89110787.2
(22) Anmeldetag: 16.08.1986
(51) Int. Cl.: A61K 9/48, A61K 9/66

(54) **Weichgelatinekapseln und Verfahren zu ihrer Herstellung**
Soft gelatin capsules and process for their preparation
Capsules de gélatine molle et leur procédé de fabrication

(30) Priorität: 20.08.1985 DE 3529694
(43) Veröffentlichungstag der Anmeldung: 18.10.1989
(62) Teilanmeldung aus: 86904825.6
(73) Patentinhaber: R.P. Scherer GmbH, D-69412 Eberbach (DE)
(72) Erfinder: Fischer, Gerhard, Dr., D-6930 Eberbach/Baden (DE)
(74) Vertreter: Werner, Hans-Karsten, Dr.Dipl.-Chem.

(56) Entgegenhaltungen:
- EP-A- 0 092 908
- FR-A- 2 116 021
- FR-A- 2 193 059
- US-A- 3 758 323

## Beschreibung

Gegenstand der Vorliegenden Erfindung sind Weichgelatinekapseln bestehend aus einer Kapselhülle und einer Kapselfüllung, wobei die Kapselhülle aus Gelatine, gegebenenfalls einem Weichmacher und weiteren Zusätzen besteht und die Kapselfüllung Wirkstoffe und/oder Diätmittel sowie gegebenenfalls weitere Zusätze und eine Flüssigkeit oder Pulver enthält. Weiterhin betrifft die Erfindung Verfahren zur Herstellung derartiger Weichgelatinekapseln.

Die Herstellung von Weichgelatinekapseln ist z.B. in Lachmann, Theory and Practice of Industrial Pharmacy, Lea and Febiger, Philadelphia, 2. Ausgabe, ausführlich beschrieben. Es wird dort ausgeführt, daß die Art der Weichkapselfüllgüter im wesentlichen auf drei Kategorien beschränkt ist, nämlich nicht wassermischbare, nicht flüchtige oder leicht flüchtige Träger wie Öle, Fette, etherische Öle, chlorierte Kohlenwasserstoffe, Ester, Ether, höhere Alkohole und organische Säuren. Diese Kategorie von Hilfs- und Wirkstoffen ist daher relativ einfach zu verkapseln und läßt einen ziemlich großen Spielraum für die Formulierung der Kapselhülle.

Die zweite Kategorie umfaßt mit Wasser mischbare, nicht flüchtige Stoffe wie Polyethylenglykole oder Emulgatoren. Diese Kategorie ist weitaus schwieriger zu verarbeiten. So beschreibt z.B. die DE-OS 33 07 353 ein Verfahren zur Herstellung von polyethylenglykolhaltigen Weichgelatinekapseln, wonach Polyethylenglykol nur dann zuverlässig verkapselt werden kann, wenn ganz bestimmte Parameter sowohl bezüglich der Kapselhülle als auch bezüglich des Kapselfüllgutes eingehalten werden.

Die Verkapselungsprobleme können damit erklärt werden, daß polyethylenglykolhaltige Füllungen in starke Wechselwirkungen mit der Kapselhülle treten und so die Lagerstabilität der Kapseln nicht mehr ohne weiteres gewährleistet ist.

Die dritte Kategorie schließlich umfaßt wassermischbare und relativ schwer flüchtige Komponenten wie z.B. Glycerin, Propylenglykol oder Benzylalkohol. Bei dieser Kategorie sind die in der Kategorie (2) beschriebenen Wechselwirkungen so stark, daß Komponenten der letzten Kategorie nur in Konzentrationen bis zu maximal 10% verkapselt werden können.

Als nicht verkapselbar gelten nach Lachmann, loc.cit. Flüssigkeiten mit mehr als 5% Wasser und niedermolekulare, organische, wasserlösliche Verbindungen, insbesondere niedere Alkohole wie Ethylalkohol, Ketone und Amine.

Besondere Probleme bereitet auch die Verarbeitung wasserlöslicher oder hygroskopischer Wirkstoffe in Gelatinekapseln, da diese ebenso die Kapselwand negativ beeinflussen.

Die US-A-2 580 683 beschreibt Gelatinekapseln, die mit hoch konzentrierter Lösungen von hygroskopischem Material, wie Glykosesirup etc., gefüllt sind, wobei die Kapselfüllung so hoch konzentriert an hygroskopischem Material ist, daß die Kapselfüllung nicht mehr in der Lage ist, die Kapselhülle zu zerstören. Weiterhin wird hierin beschrieben, daß die Konzentration an hygroskopischem Material der Kapselfüllung verringert werden kann, wenn in der Kapselhülle auf ein Teil Gelatine zusätzlich ein Teil Zucker eingearbeitet wird. Durch diese Maßnahme ist es möglich, etwas niedrigerviskose Kapselfüllungen zu verarbeiten. Auf alle Fälle muß jedoch eine sehr hohe Konzentration an hygroskopischem Material in der Kapselfüllung vorhanden sein, ohne daß dies durch konkrete Zahlenangaben festgelegt ist.

Die EP-A-0 120 248 beschreibt polyethylenglykolhaltige Weichgelatinekapseln und Verfahren zu ihrer Herstellung. Dabei werden übliche Kapselhüllen eingesetzt. Die Kapselfüllung besteht aus Lösungen oder Suspensionen des Wirkstoffes in Polyethylenglykol, von dem mindestens 50 Gew.-% ein mittleres Molekulargewicht von 600 aufweist. Zusätzlich enthält die Kapselfüllung Glycerin und/oder 1,2-Propylenglykol.

FR-A-2 116 021 beschreibt Formkörper aus Gelatine, die im wesentlichen aus einem innigen Gemisch von Gelatine und Amylose und/oder Amylitol bestehen, wobei der Polymerisationsgrad der Amylose oder des Amyiitols weniger als 50 beträgt. Derartige Gelatineprodukte lassen sich gut zu Folien und gegebenenfalls auch Hartgelatinekapseln verarbeiten, jedoch nicht zu Weichgelatinekapseln. Versuche, mit derartigen wasserlöslichen und stark mit Wasser quellenden Stärkederivaten Weichgelatinekapseln herzustellen, sind stets daran gescheitert, daß die Nähte nicht halten und somit keine brauchbaren Weichgelatinekapseln hergestellt werden können.

Aus der nicht vorpublizierten EP-A-0 199 034 ist die Verkapselung von Honig, Invertzucker oder Zuckersirup in normalen Weichgelatinehüllen bekannt, indem der Kapselfüllung 2 bis 20 Gew.-% Glycerin beigemischt wurden. Während bei sonstigen Kapselfüllungen derartig hohe Mengen von Glycerin zur Zerstörung der Kapselhülle führen, wird dieses Glycerin offensichtlich durch Honig, Invertzucker oder Zuckersirup so an die Kapselfüllung gebunden, daß es keinen schädlichen Einfluß auf die Kapselhülle ausüben kann. Dennoch ist es nötig, die so hergestellten Weichgelatinekapseln geschützt zu lagern, da sie sonst nur einige Wochen haltbar sind.

Die Erfindung der Stammanmeldung 86 904 825 hat sich die Aufgabe gestellt, diese dritte, bisher nicht verkapselbare Gruppe von Substanzen zu verkapseln, d.h. Kapselfüllungen herzustellen, welche mindestens einen mit Wasser mischbaren, wasserlöslichen, wasserempfindlichen oder hydrophilen, leicht flüchtigen Stoff enthalten. Weiterhin hat sie sich die Aufgabe gestellt, nur schwierig verkapselbare Substanzen der zweiten Kategorie leichter zu verkapseln.

Dabei wurde überraschenderweise gefunden, daß diese Aufgabe dadurch gelöst werden kann, daß
a) die Kapselhülle als Zusatz ein Mittel enthält, welches in reiner Form mindestens 10 Gew.-% seines Eigengewichts an Wasser aufnehmen kann, ohne sich äußerlich zu verändern, wobei dieses Mittel aus einem oder mehreren Stoffen aus der Gruppe der Stärken, Stärkederivate, Zellulosen, Zellulosederivate, Milchpulver, nicht hygroskopischer Oligosaccharide, Magnesiumtrisilikat, Siliciumdioxid besteht und
b) die Kapselfüllung mindestens einen mit Wasser mischbaren, wasserlöslichen, wasserempfindlichen oder hydrophilen, leicht flüchtigen Stoff enthält.

Mit Hilfe dieser Zusätze zur Kapselhülle ist es erstmals möglich, Kapselfüllungen einzusetzen, die einen oder mehrere Stoffe enthalten aus der Gruppe der niederen Alkohole, Ether, Ester, Alkanale, Alkanone, Polyole und Amine. Weiterhin ist es möglich, Kapselfüllungen herzustellen, welche eine oder mehrere Stoffe enthalten aus der Gruppe der konzentrierten wässrigen Zuckerlösungen, Stärkesirupe, hydrierten Stärkesirupe, Zuckerderivatlösungen und Honig. Schließlich ist es möglich, nur schwierig verkapselbare Substanzen wie mit Wasser mischbare, nicht flüchtige Stoffe wie Polyethylenglykole oder Emulgatoren problemlos zu verkapseln.

Von besonderer Bedeutung sind Weichgelatinekapseln, wie sie insbesondere im pharmazeutischen Sektor zum Einsatz kommen und die unzerkaut geschluckt werden sollen. Es lassen sich aber auch weiche Kaukapseln herstellen, die insbesondere im Lebensmittel- und Diätbereich zum Einsatz kommen können. Prinzipiell ist es aber auch möglich, mit Hilfe der Zusätze zur Kapselhülle stabile Hartgelatinekapseln herzustellen, die trotz der ungewöhnlichen Kapselfüllungen lagerbeständig sind.

Es wurde somit festgestellt, daß die Wechselwirkung zwischen Kapselhülle und Umwelt erheblich zurückgedrängt wird. So sind Kapseln im allgemeinen nur bei 15 bis 25°C und einer relativen Luftfeuchte unter 60°C dauerhaft lagerfähig, da die Kapseln durch Aufnahme von Luftfeuchtigkeit weich werden und/oder beginnen zusammenzukleben. Die neuen Kapseln sind weitaus weniger anfällig gegen Verkleben und Weichwerden, was durch eine verminderte Aufnahme von Wasser aus der Umgebung erklärt werden könnte.

Diese Eigenschaften der neuen Kapselhülle sind somit nicht nur bedeutungsvoll für Kapselfüllungen gemäß der Stammanmeldung 86 904 825, sondern auch für Kapselfüllungen, die auch schon früher ohne weiteres verkapselt werden konnten.

Gegenstand der vorliegenden Erfindung sind somit Weichgelatinekapseln bestehend aus
a) einer Kapselhülle und
b) einer Kapselfüllung,
wobei die Kapselhülle aus Gelatine, gegebenenfalls einem Weichmacher und weiteren Zusätzen besteht und die Kapselfüllung Wirkstoffe und/oder Diätmittel sowie gegebenenfalls weitere Zusätze und eine Flüssigkeit oder Pulver enthält, dadurch gekennzeichnet, daß
a) die Kapselhülle als Zusatz ein Mittel enthält, welches in reiner Form mindestens 10 Gew.-% seines Eigengewichts an Wasser aufnehmen kann, ohne sich äußerlich zu verändern, wobei dieses Mittel aus einem oder mehreren Stoffen aus der Gruppe der Stärken, Stärkederivate, Zellulosen, Zellulosederivate, Milchpulver, nicht hygroskopischer Oligosaccharide, Magnesiumtrisilikat, Siliciumdioxid besteht und
b) die Kapselfüllung einen oder mehrere Stoffe enthält aus der Gruppe der Pflanzenöle, halbsynthetischen oder synthetischen Öle, Fettderivate, tierischen Fette oder Öle, Lecithine und/oder pharmazeutisch verwendbaren Emulgatoren.

Ein weiterer Gegenstand der Erfindung ist das Verfahren zur Herstellung von derartigen Weichgelatinekapseln, dadurch gekennzeichnet, daß
a) dem Gelatinesud als Zusatz eine Suspension oder Lösung eines Mittels zugegeben wird, welches in reiner Form mindestens 10 Gew.-% seines Eigengewichts an Wasser aufnehmen kann, ohne sich äußerlich zu verändern, wobei dieses Mittel aus einem oder mehreren Stoffen aus der Gruppe der Stärken, Stärkederivate, Zellulosen, Zellulosederivate, Milchpulver, nicht hygroskopischer Oligosaccharide, Magnesiumtrisilikat, Siliciumdixoid besteht und
b) als Kapselfüllung einen oder mehrere Stoffe enthält aus der Gruppe der Pflanzenöle, halbsynthetischen oder synthetischen Öle, Fettderivate, tierischen Fette oder Öle, Lecithine und/oder pharmazeutisch verwendbaren Emulgatoren.

Es ist bisher noch völlig ungeklärt, wieso der erfindungsgemäße Zusatz von Mitteln, welche in reiner Form mindestens 10 Gew.-% ihres Eigengewichts an Wasser aufnehmen können, ohne sich äußerlich zu verändern, die Eigenschaften der Kapselhülle derartig verändert, daß bisher als nicht verkapselbar geltende Kapselfüllungen zum Einsatz kommen können. Ungeklärt ist auch jetzt noch, warum die neuen Kapseln weniger anfällig sind gegen Verkleben und Weichwerden.

Die Wahl und Konzentration dieses Mittels richten sich in gewissem Maße nach der speziellen Kapselfüllung. Ebenso ist die Art der Einarbeitung dieser Mittel in die Gelatinehülle bis zu einem gewissen Grade produktabhängig. Dennoch sprechen alle bisherigen Ergebnisse dafür, daß es allein durch den Zusatz dieser Mittel gelingt, die Kapselhülle gegen Einflüsse aus der Kapselfüllung und der Umwelt zu inertisieren und die Durchlässigkeit für alle wasserlöslichen, hydrophilen und leicht flüchtigen Stoffe zu verringern. Erstaunlicherweise können diese Mittel bei der Verarbeitung entweder als Dispersion oder sogar als Lösung dem Gelatinesud zugegeben werden, d.h. also in einer Form, in der sie aufgrund der großen Wassermenge durchaus in der Lage sind, sich äußerlich zu verändern. In der endgültigen Kapselhülle scheinen sie eine Art Pufferwirkung auf den Wassergehalt der Hülle und der Kapselfüllung auszuüben, ohne daß jedoch diese Wirkung eine Erklärung dafür liefern kann, warum hierdurch erfindungsgemäß stabile und brauchbare Kapseln entstehen.

Die erfindungsgemäßen Zusätze zu Kapselhülle können aus den verschiedensten Stoffen ausgewählt sein. Besonders bewährt haben sich zunächst Stärken und Stärkederivate sowie Zellulosen und Zellulosederivate. Hierbei handelt es sich somit um native Kartoffel-, Mais-, Weizen- oder Tapiokastärke. Als Stärkederivate kommen physikalisch behandelte, modifizierte Stärken, oxidierte, hydrolysierte, enzymbehandelte, vernetzte, veresterte oder veretherte Stärken in Frage. Als Zellulose ist insbesondere mikrokristalline Zellulose verwendbar sowie Zellulosederivate, welche die erfindungsgemäßen Bedingungen bezüglich des Verhaltens gegenüber Wasser erfüllen.

Erstaunlicherweise sind auch Milchpulver und nicht hygroskopische Oligosaccharide geeignet. Aber auch anorganische Stoffe wie Magnesiumtrisilikat und kolloidales Siliciumdioxid erfüllen die erfindungsgemäße Aufgabe.

Einige dieser Stoffe sind in der Lage, wesentlich mehr als 10 Gew.-% ihres Eigengewichts an Wasser aufzunehmen, ohne sich äußerlich zu verändern. Andere Mittel dieser Stoffgruppe werden von größeren Mengen Wasser sogar klar gelöst. Dennoch erfüllen sie die erfindungsgemäße Aufgabe bezüglich der Verbesserung der Kapselhülle.

Zur Durchführung des erfindungsgemäßen Verfahrens zur Herstellung von Gelatinekapseln werden die Mittel in Form einer Suspension oder Lösung dem Gelatinesud zugegeben. Nach der üblichen Füllung und Trocknung der Kapseln entstehen unmittelbar brauchbare Produkte.

## Patentansprüche

1. Weichgelatinekapseln bestehend aus
a) einer Kapselhülle und
b) einer Kapselfüllung,
wobei die Kapselhülle aus Gelatine, gegebenenfalls einem Weichmacher und weiteren Zusätzen besteht und die Kapselfüllung Wirkstoffe und/oder Diätmittel sowie gegebenenfalls weitere Zusätze und eine Flüssigkeit oder Pulver enthält, dadurch gekennzeichnet, daß
a) die Kapselhülle als Zusatz ein Mittel enthält, welches in reiner Form mindestens 10 Gew.-% seines Eigengewichts an Wasser aufnehmen kann, ohne sich äußerlich zu verändern, wobei dieses Mittel aus einem oder mehreren Stoffen aus der Gruppe der Stärken, Stärkederivate, Zellulosen, Zellulosederivate, Milchpulver, nicht hygroskopischer Oligosaccharide, Magnesiumtrisilikat, Siliciumdioxid besteht und
b) die Kapselfüllung einen oder mehrere Stoffe enthält aus der Gruppe der Pflanzenöle, halbsynthetischen oder synthetischen Öle, Fettderivate, tierischen Fette oder Öle, Lecithine und/oder pharmazeutisch verwendbaren Emulgatoren.

2. Verfahren zur Herstellung von Weichgelatinekapseln gemäß Anspruch 1, dadurch gekennzeichnet, daß
a) dem Gelatinesud als Zusatz eine Suspension oder Lösung eines Mittels zugegeben wird, welches in reiner Form mindestens 10 Gew.-% seines Eigengewichts an Wasser aufnehmen kann, ohne sich äußerlich zu verändern, wobei dieses Mittel aus einem oder mehreren Stoffen aus der Gruppe der Stärken, Stärkederivate, Zellulosen, Zellulosederivate, Milchpulver, nicht hygroskopischer Oligosaccharide, Magnesiumtrisilikat, Siliciumdixoid besteht und
b) als Kapselfüllung einen oder mehrere Stoffe enthält aus der Gruppe der Pflanzenöle, halbsynthetischen oder synthetischen Öle, Fettderivate, tierischen Fette oder Öle, Lecithine und/oder pharmazeutisch verwendbaren Emulgatoren.

## Claims

1. Soft gelatin capsules consisting of
a) a capsule sheath and
b) a capsule filling,
the capsule sheath consisting of gelatin, optionally a plasticizer and further additives and the capsule filling containing active ingredients and/or dietary agents and optionally further additives and a liquid or a powder,
characterized in that
a) the capsule sheath contains, as an additive, at least 1% by weight of an agent which in its pure state is capable of absorbing water in an amount of at least 10% of its own weight without undergoing any external change, said agent consisting of one or more substances from the group of the starches, starch derivatives, celluloses, cellulose derivatives, milk powder, non-hygroscopic oligosaccharides, magnesium trisilicate, silicon dioxide, and
b) the capsule filling contains one or more substances from the group of vegetable oils, semi-synthetic or synthetic oils, fat derivatives, animal fats or oils, lecithins and/or pharmaceutically usable emulsifiers.

2. A process for the preparation of soft gelatin capsules according to claim 1, characterized in that
a) a suspension or solution of at least 1% by weight of an agent is added, as an additive, to the gelatin stock, which agent, in its pure state, is capable of absorbing water in an amount of at least 10% of its own weight without undergoing any external change, said agent consisting of one or more substances from the group of the starches, starch derivatives, celluloses, cellulose derivatives, milk powder, nonhygroscopic oligosaccharides, magnesium trisilicate, silicon dioxide, and
b) the capsule filling contains one or more substances from the group of vegetable oils, semi-synthetic or synthetic oils, fat derivatives, animal fats or oils, lecithins and/or pharmaceutically usable emulsifiers.

## Revendications

1. Capsules de gélatine molle constituées de
a) une enveloppe de capsule et
b) un remplissage de capsule,
où l'enveloppe de capsule est constituée de gélatine, éventuellement d'un plastifiant et d'autres additifs et le remplissage de capsule comporte des matières actives et/ou un produit dc régime ainsi éventuellement que d'autres additifs et un liquide ou une poudre, caractérisées en ce que
a) l'enveloppe de capsule contient comme additif un agent qui, dans sa forme pure, peut absorber au moins 10 % en poids ce son propre poids d'eau, sans se modifier extérieurement, cet agent étant constitué d'une ou plusieurs matières du groupe formé par les amidons, les dérivés d'amidon, les celluloses, les dérivés cellulosiques, les poudres de lait, les oligosaccharides non hydroscopiques, le trisilicate de magnésium, la silice, et
b) la remplissage de capsule comporte une ou plusieurs matières du groupe formé par les huiles végétales, les huiles semi-synthétiques ou synthétiques, les dérivés de graisses, les graisses ou huiles animales, la lécithine et/ou les agents émulsionnants utilisables en pharmacie.

2. Procédé pour préparer des capsules de gélatine molle selon la revendication 1, caractérisé en ce que
a) on ajoute comme additif à la décoction de gélatine une suspension ou une solution d'un agent qui, dans sa forme pure, peut absorber au moins 10 % en poids de son propre poids d'eau, sans se modifier extérieurement, cet agent étant constitué d'une ou plusieurs matières du groupe formé par les amidons, les dérivés d'amidon, les celluloses, les dérivés cellulosiques, les poudres de lait, les oligosaccharides non hydroscopiques, le trisilicate de magnésium, la silice, et
b) on utilise un remplissage de capsule qui contient une ou plusieurs matières du groupe formé par les huiles végétales, les huiles semi-synthétiques ou synthétiques, les dérivés de graisses, les graisses ou huiles animales, la lécithine et/ou les agents émulsionnants utilisables en pharmacie.
